# EUROPEAN PATENT APPLICATION

(11) **EP 1 172 360 A1**
(43) Date of publication of application: **16.01.2002**
(21) Application number: 00917353.5
(22) Date of filing: 19.04.2000
(51) Int. Cl.: C07D 221/12, A61K 31/473, A61P 25/28, A61P 25/16, A61P 25/14, A61P 25/02

(54) **NEUROTROPHIN POTENTIATORS**

(30) Priority: 19.04.1999 JP 11076699
(71) Applicant: Sankyo Company, Limited, Tokyo 103-8426 (JP)
(72) Inventor: ISONO, Fujio Sankyo Company, Limited, Tokyo 140-8710 (JP); FUJII, Miyuki Sankyo Company, Limited, Tokyo 140-8710 (JP); AOYAGI, Atsushi Sankyo Company, Limited, Tokyo 140-8710 (JP)
(74) Representative: Gibson, Christian John Robert
(86) International application number: JP0002534
(87) International publication number: WO0063179

(57) **Abstract**

A potentiator for neurotrophin effect comprising a 6(5H)-phenanthridinone derivative represented by the following formula (I): 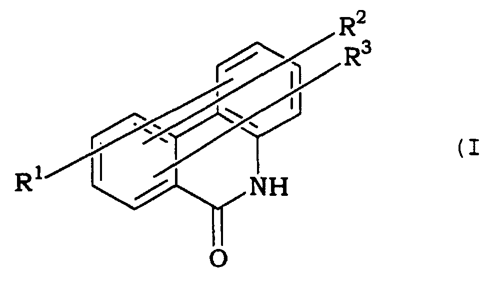 (wherein R¹ and R² are the same or different and each independently represents a hydrogen atom or a halogen atom and R³ represents an amino group) or a pharmacologically acceptable salt thereof is useful as a preventive or therapeutic agent for neurodegenerative diseases.

## Description

### Technical Field

The present invention relates to a potentiator for neurotrophin effect which contains a 6(5H)-phenanthridinone derivative or a pharmacologically acceptable salt thereof, an agent for the prevention or treatment of neurodegenerative diseases containing the potentiator for neurotrophin effect and use of a 6(5H)-phenanthridinone derivative or a pharmacologically acceptable salt thereof for preparing a composition for the prevention or remedy of neurodegenerative diseases.

### Background Art

Neurodegenerative diseases due to neurodegeneration or cell death include Alzheimer-type dementia, Parkinson's disease, amyotrophic lateral sclerosis, Huntington's chorea and some peripheral sensory neuropathies. For example, in Alzheimer-type dementia, the degeneration or loss of basal forebrain cholinergic neurons or pyramidal neurons in the hippocampus or the cerebral cortex are recognized and are considered to cause the exhibition of dementia symptoms. In Parkinson's disease, a selective neurodegeneration or loss of midbrain dopaminergic neurons is believed to be a cause of the movement disorder. In amyotrophic lateral sclerosis, Huntington's chorea or peripheral sensory neuropathy in patients with diabetes mellitus or cancer patients treated with carcinostatic agents, degeneration of motor neurons, strial neurons and sensory neurons in the spinal cord are presumed to be causes of these diseases, respectively. At present, there are no agents to stop the progress of neurodegeneration. Under the present situation, only a replacement therapy is conducted to relieve symptoms temporarily.

In particular, an increase in the number of patients suffering from Alzheimer-type dementia has recently been a serious social problem, but as a therapeutic agent for it, only acetylcholineesterase inhibitors are therapeutically used. These agents relieve the symptoms temporarily, but cannot retard the progress of the disease. Cholinesterase inhibitors, therefore, are considered to be effective, only for a short term, for patients with Alzheimer-type dementia. In addition, cholinesterase inhibitors also act on the peripheral nervous system so that some serious adverse events may occur. Accordingly, they are not suitable therapeutic agents for patients with Alzheimer-type dementia who require long-term treatment. In WO 98/27975, it is described that 6(5H)-phenanthridinone, 2-nitro-6(5H)-phenanthridinone, etc. are useful for Alzheimer-type dementia, because they prolong the survival time of cells and potentiate the proliferation activity of the cells due to inhibition of Poly(ADP-ripose)polymerase. However, the effects of the compound on neurons in the brain, which do not proliferate, are not described. Thus it is unclear why the compounds are useful for Alzheimer-type dementia. Although dopamine substitution therapy by L-DOPA has marked therapeutic effects on Parkinson's disease, long-term L-DOPA therapy causes various adverse events such as involuntary movement or psychic symptoms. Amyotrophic lateral sclerosis, Huntington's chorea and peripheral sensory neuropathy have no effective therapeutic agents so far.

Under these circumstances recent attention has been focused on neurotrophic factors as novel therapeutic agents for neurodegenerative diseases [Annual Review of Pharmacology and Toxicology, 37, 239 (1997)]. Neurotrophic factors are protein groups having an important role for exhibition of the neuronal functions *in vivo* and they can contribute to the recovery of damaged neuronal functions. Among such neurotrophic factors called "neurotrophins" involve nerve growth factor, brain-derived neurotrophic factor, neurotrophin-3 and neurotrophin-4/5 (which will hereinafter be abbreviated as "NGF", "BDNF", "NT-3" and "NT-4/5", respectively) and they play an important role for survival and maintenance of the neuronal functions.

NGF acts on sensory neurons of the dorsal root ganglion in the spinal cord and sympathetic neurons in the peripheral nervous system, as well as cholinergic neurons in the forebrain-basal nucleus and the striatum. BDNF acts widely on various neurons involving sensory neurons in the dorsal root ganglion of the spinal cord, spinal motor neurons, cholinergic and GABA-energic neurons in the forebrain basal nucleus, midbrain dopaminergic neurons, serotonergic neurons in the raphe nucleus, pyramidal neurons in the hippocampus or cerebral-cortex, cerebellar granular cells, and neurons in the retina. NT-3 acts on sensory neurons, motor neurons and pyramidal neurons in the hippocampus or the cerebral-cortex. NT-4/5 acts on sympathetic neurons and sensory neurons in the dorsal root ganglion of the spinal cord.

Since it has been revealed that neurotrophins actually suppress neurodegeneration and restore the neuronal function in various animal models of neurodegenerative diseases, they are considered to be useful as therapeutic agents for the neurodegenerative diseases described above. For example, NGF suppresses degeneration of damaged cholinergic neurons or cholinergic neurons in aged animals and improves memory and learning tasks in animals [The Journal of Neuroscience, 14, 4815 (1994)]. Thus NGF is considered to be useful as a therapeutic agent for Alzheimer-type dementia or with vascular dementia. BDNF recovers neuronal functions of cholinergic neurons [The Journal of Neuroscience, 12, 4391 (1992)], dopaminergic neurons [Proceedings of the National Academy of Sciences of the United States of America, 89, 11347 (1992)] and motoneurons which have been damaged [Nature, 360, 753 (1992)]. Further, BDNF is believed to be associated with long-term potentiation in the hippocampus and the cerebral cortex which is thought to be a basic process of memory [Proceedings of the National Academy of Sciences of the United States of America, 92, 8856 (1995)], Thus BDNF is considered to be useful as a therapeutic agent for dementia, Parkinson's disease, spinal motor neuron disorders or amyotrophic lateral sclerosis. Moreover, since NGF and NT-3 suppress degeneration of peripheral sensory neurons and restore their functions [Annals of Neurology, 29, 87 (1991), ibid., 38, 20 (1995)], they are considered to be useful as therapeutic agents for peripheral sensory neuropathy in patients with diabetes mellitus or in patients with cancer to whom carcinostatic agents have been administered.

Further, since NGB or BDNF reduces neuronal damages in the animal stoke model [The Journal of Neuroscience, 11, 2914 (1991), Neurosurgery, 34, 323 (1994)], they may be useful as a protective agents following cerebral ischemia and therapeutic agents for sequelae of the disease.

The application of neurotrophins is, however, limited as they are proteins. Direct administration into the cerebral ventricles is required in order to make them act on the central nervous system. In addition, since neurotrophins are highly active, neurotrophins when administered systemically may cause adverse events when acting on sites inherently free from a large amount of neurotrophin. For example it has been reported that treatment of a patient by intracerebraventicular injection of NGF had to be halted due to the patient experiencing severe pain. This is presumably due to hyperactivation of sensory neurons in the spinal cord which are not target sites.

In order to overcome these problems, steroids, catechols and polyene compounds are known to synthesize and/or secrete NGF. It is also reported that these compounds improved learning and memory function in animal models. However the targets of NGF production enhancers are non-neurons and they may have a high risk of affecting tissues other than the nervous system, for example, immunological tissues. Moreover, some of these substances stimulate NGF production at close concentrations to those which induce cytotoxicity. Therefore these agents are not practical. In addition, when they are administered systemically, NGF may be produced at sites where inherently free of NGF and possibly cause adverse events similar to NGF itself.

### Disclosure of the Invention

With a view to developing a novel anti-neurodegenerative agent, the present inventors carried out an extensive investigation on the pharmacological activity of various 6(5H)-phenanthridinone derivatives for long years. As a result, it has been found that a 6(5H)-phenanthridinone derivative having a specific structure has excellent neurotrophin-action enhancing activity and is useful as an anti-neurodegenerative agent [a preventive or therapeutic agent (particularly, a therapeutic agent) for Alzheimer-type dementia, Parkinson's disease, amyotrophic lateral sclerosis, Huntington's chorea and peripheral sensory neuropathy (particularly, Alzheimer-type dementia), or a preventive agent for apoptosis of neurons after cerebral ischemia or a therapeutic agent for the sequelae of it], leading to the completion of the present invention.

The potentiator for neurotrophin effect of the present invention acts on the target neurons of neurotrophin and reinforces the action of neurotrophin, which is a novel action mechanism. Administration of the potentiator for neurotrophin effect of the present invention to the living body reinforces the action of neurotrophin present *in vivo*, thereby making it possible to exhibit effects for treating neurodegenerative diseases as described above or for inhibiting apoptosis of neurons after cerebral ischemia. Since such an enhancer acts only on a site *in vivo* where neurotrophin actually functions, there is less possibility of side effects, different from NGF or NGF synthesis enhancer. In addition, by administration of the potentiator for neurotrophin effect of the present invention together with a neurotrophin or a neurotrophin production enhancer in an amount small enough not to cause side effects to the living body, it is possible to exhibit remedial effects while reinforcing only the target action.

The present invention provides a potentiator for neurotrophin effect containing a 6(5H)-phenanthridinone derivative or pharmacologically acceptable salt thereof, a composition for the prevention or remedy of neurodegenerative diseases containing the potentiator for neurotrophin effect and use of a 6(5H)-phenanthridinone derivative or pharmacologically acceptable salt thereof for preparing a composition for the prevention or remedy of neurodegenerative diseases.

The potentiator for neurotrophin effect of the present invention comprises a 6(5H)-phenanthridinone derivative represented by the following formula (I): 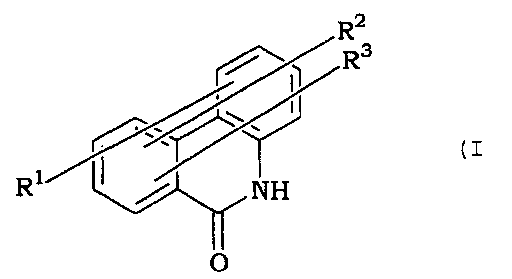 or a pharmacologically acceptable salt thereof.

In the above-described formula, R¹ and R² are the same or different and each independently represents a hydrogen atom or a halogen atom and R³ represents an amino group.

The "halogen atom" in the definition of R¹ or R² may mean, for example, a fluorine, chlorine, bromine or iodine atom, preferably a fluorine, chlorine or bromine atom, more preferably a chlorine or bromine atom, especially a chlorine atom.

The compound of formula (I) serving as an active ingredient for the potentiator for neurotrophin effect of the present invention can be easily converted into its pharmacologically acceptable salt by treating with an acid. Examples of such salts include inorganic acid salts such as hydrochloride, sulfate, nitrate and phosphate, and organic acid salts such as acetate, propionate, butyrate, benzoate, oxalate, malonate, succinate, maleate, fumarate, tartrate, citrate, methanesulfonate, ethanesulfonate, benzenesulfonate and p-toluenesulfonate. Of these, the hydrochloride, sulfate, nitrate, oxalate, succinate, fumarate and methanesulfonate salts are preferred, of which the hydrochloride salt is particularly preferred.

The compound of formula (I) serving as an active ingredient for the potentiator for neurotrophin effect of the present invention may form a hydrate by absorbing water or having adsorbed water attached thereto when allowed to stand in the air or to recrystallize. A potentiator for neurotrophin effect containing such a water-containing compound salt also forms part of in the present invention.

In the present invention, preferred is a potentiator for neurotrophin effects containing a compound represented by the following formula (la): 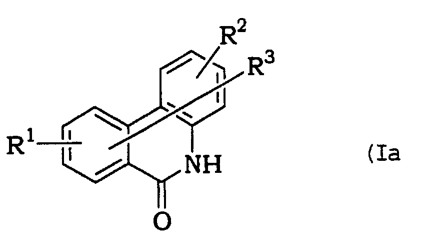 or a pharmacologically acceptable salt thereof, of which a potentiator for neurotrophin effects containing a compound represented by the following formula (Ib): 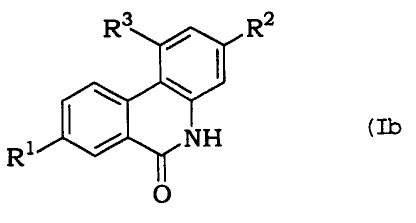 or a pharmacologically acceptable salts thereof are more preferred.

In the present invention, preferred examples include a potentiator for neurotrophin effects containing a compound of formula (I) or a pharmacologically acceptable salt thereof, wherein in the formula (I):
(1) R¹ and R² are the same or different and each independently represents a hydrogen atom, a chlorine atom or a bromine atom,
(2) R¹ and R² each independently represents a chlorine atom or a bromine atom, and
(3) R¹ and R² each represents a chlorine atom. They are preferred in the order or mention from (1) to (3).

In the potentiator for neurotrophin effect of the present invention containing the compound of formula (I) or a pharmacologically acceptable salt thereof, compounds as described below in the table can be mentioned as preferred active ingredients. It should however be borne in mind that the present invention is not limited to or by the potentiator for neurotrophin effect containing any one of these compounds or pharmacologically acceptable salts thereof.

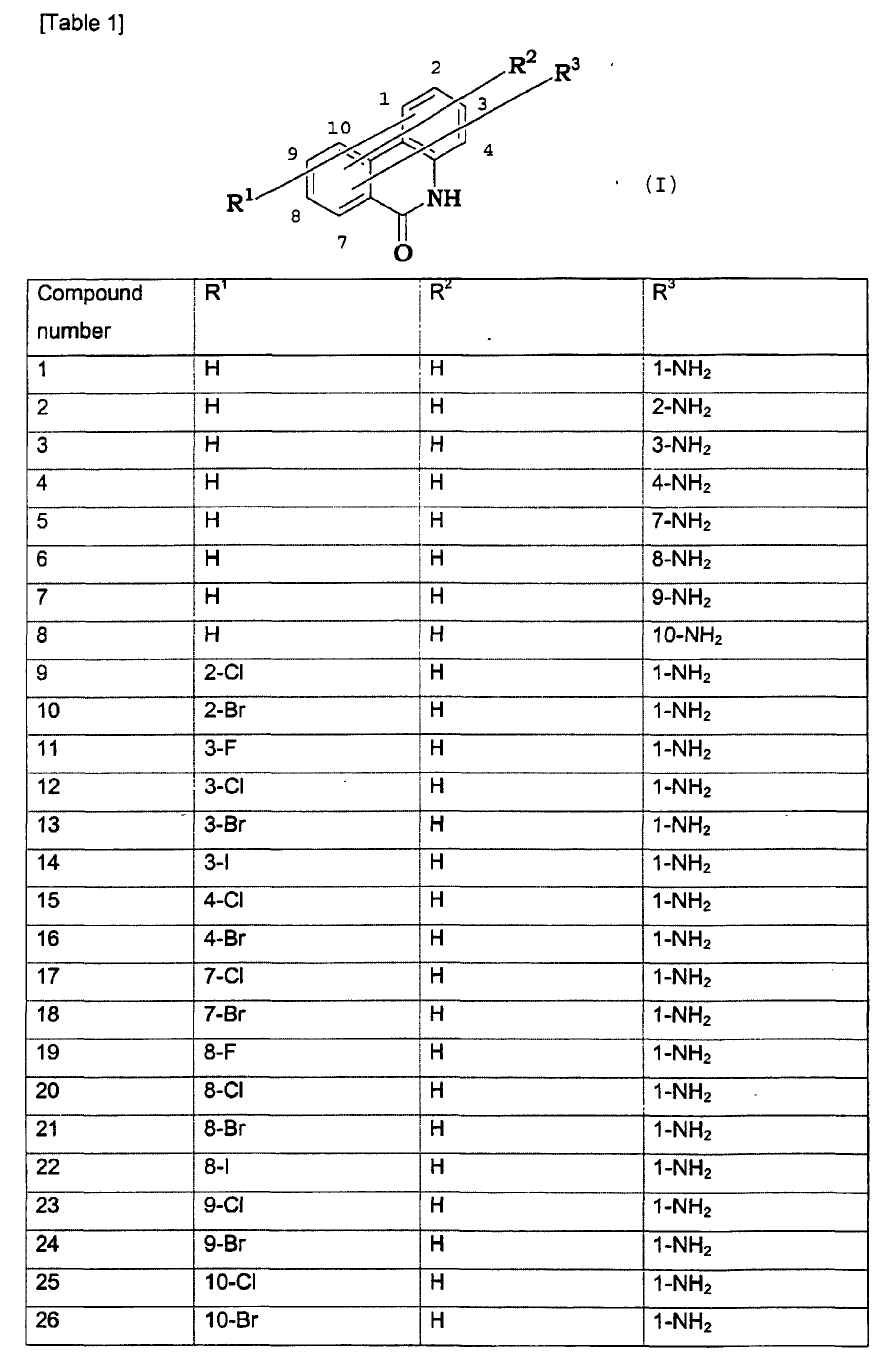

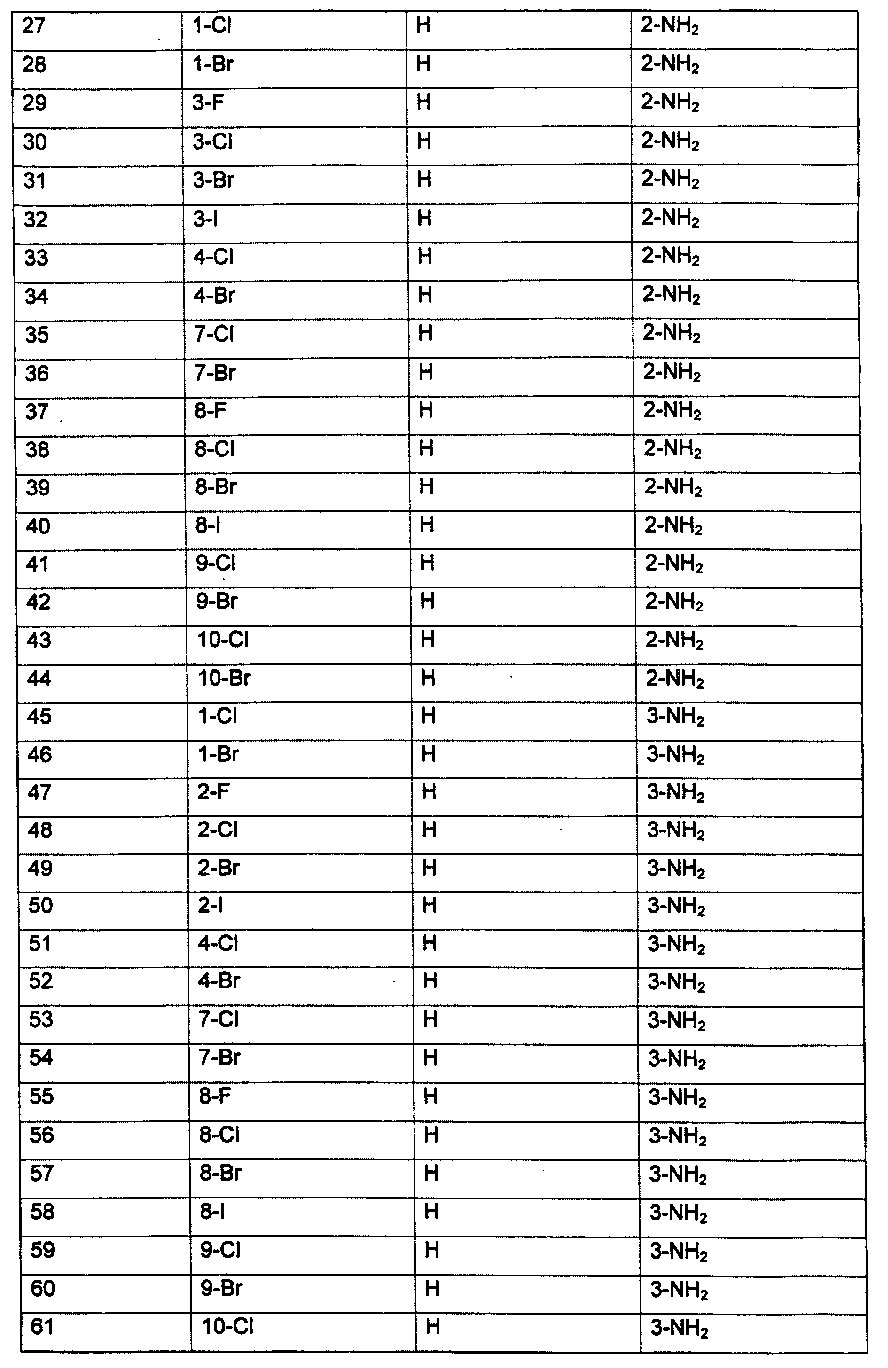

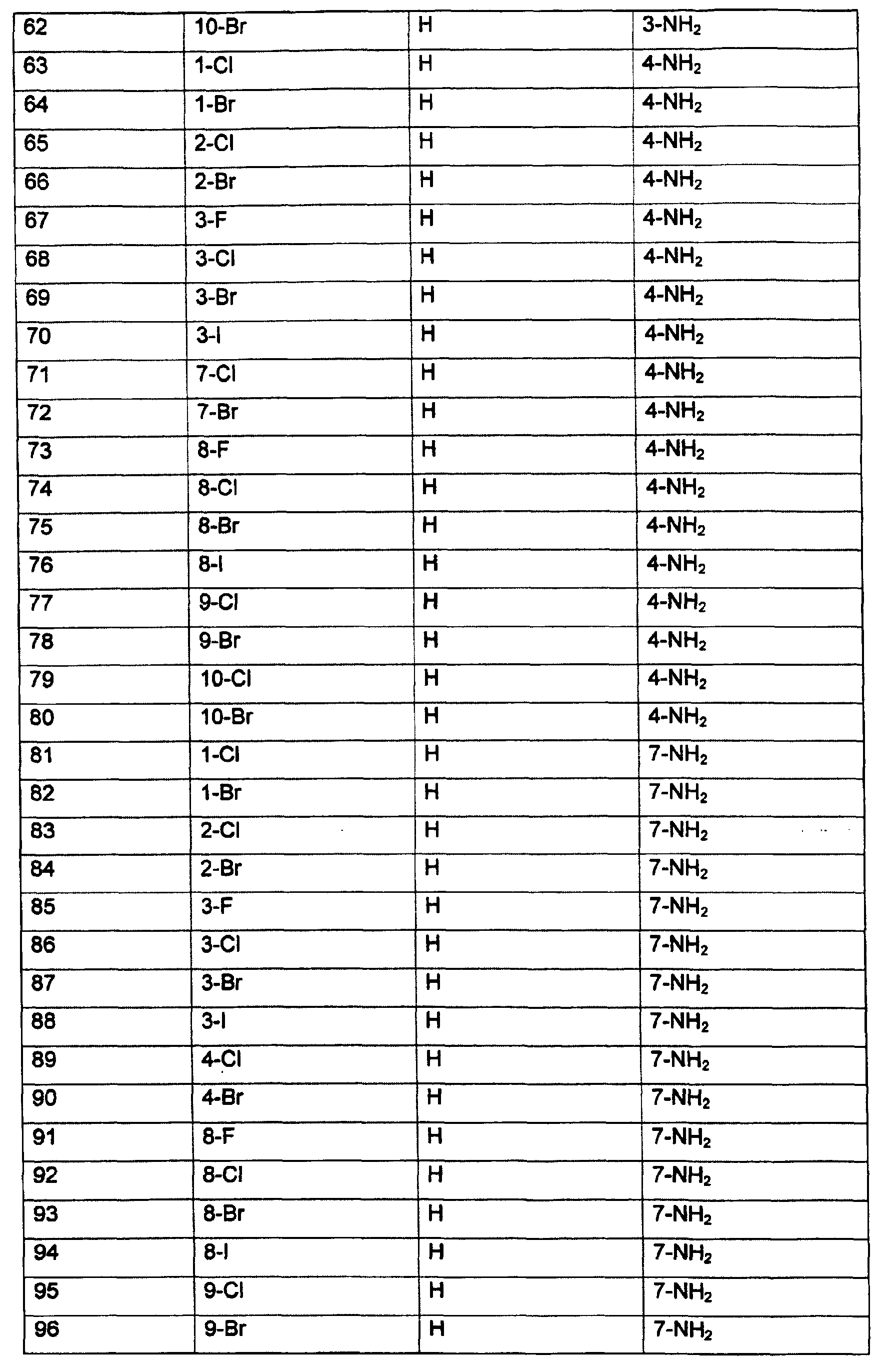

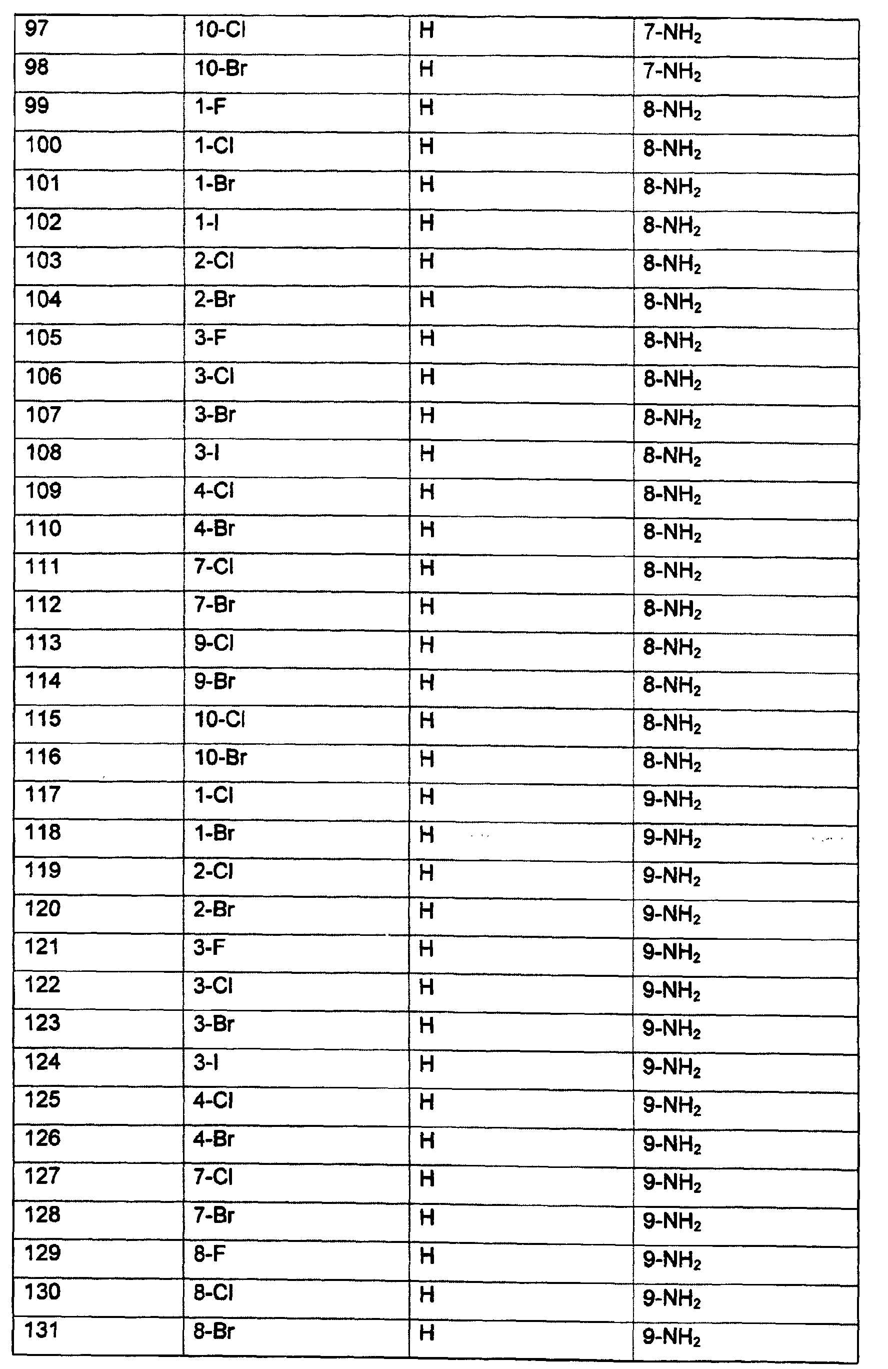

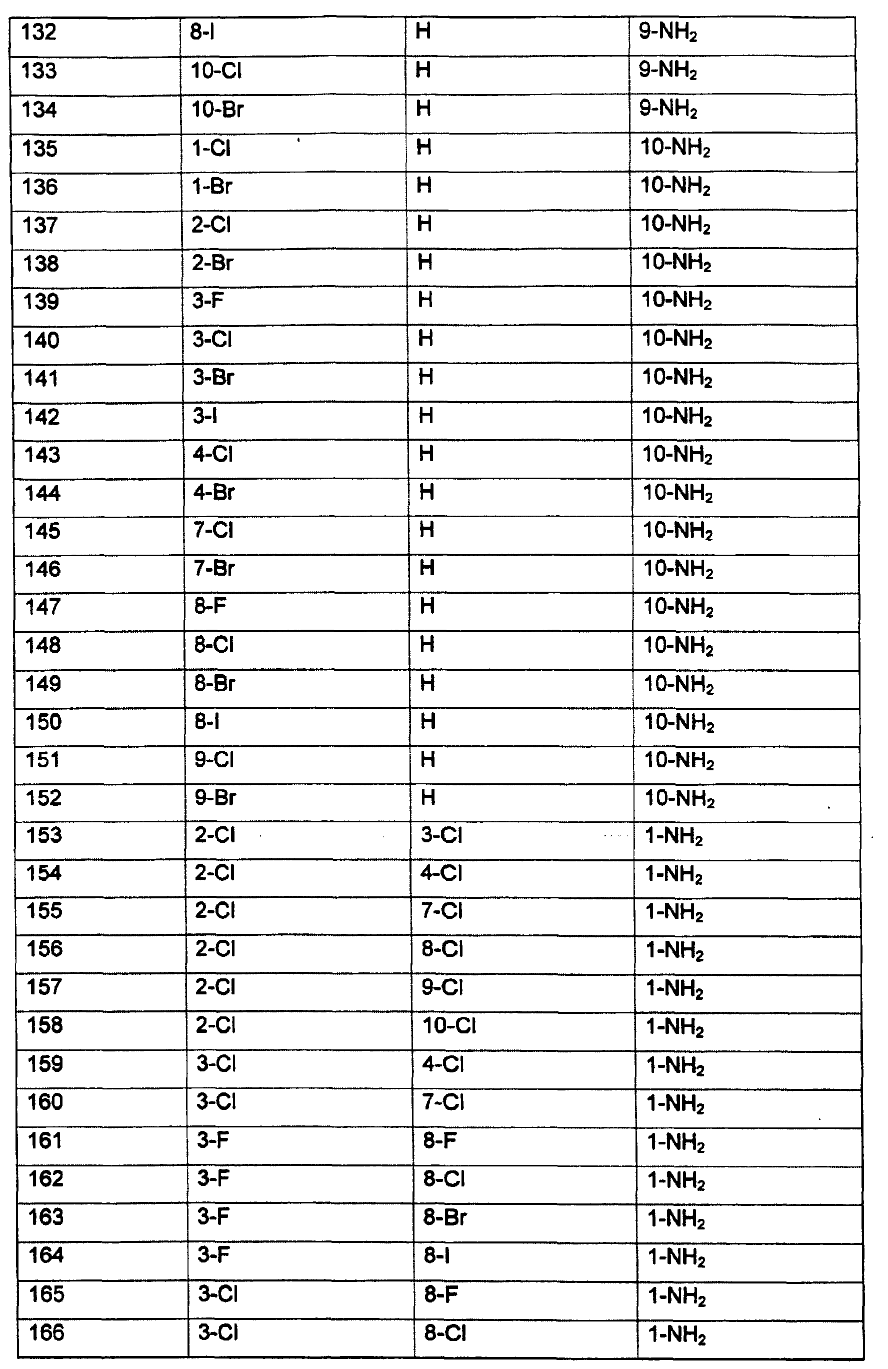

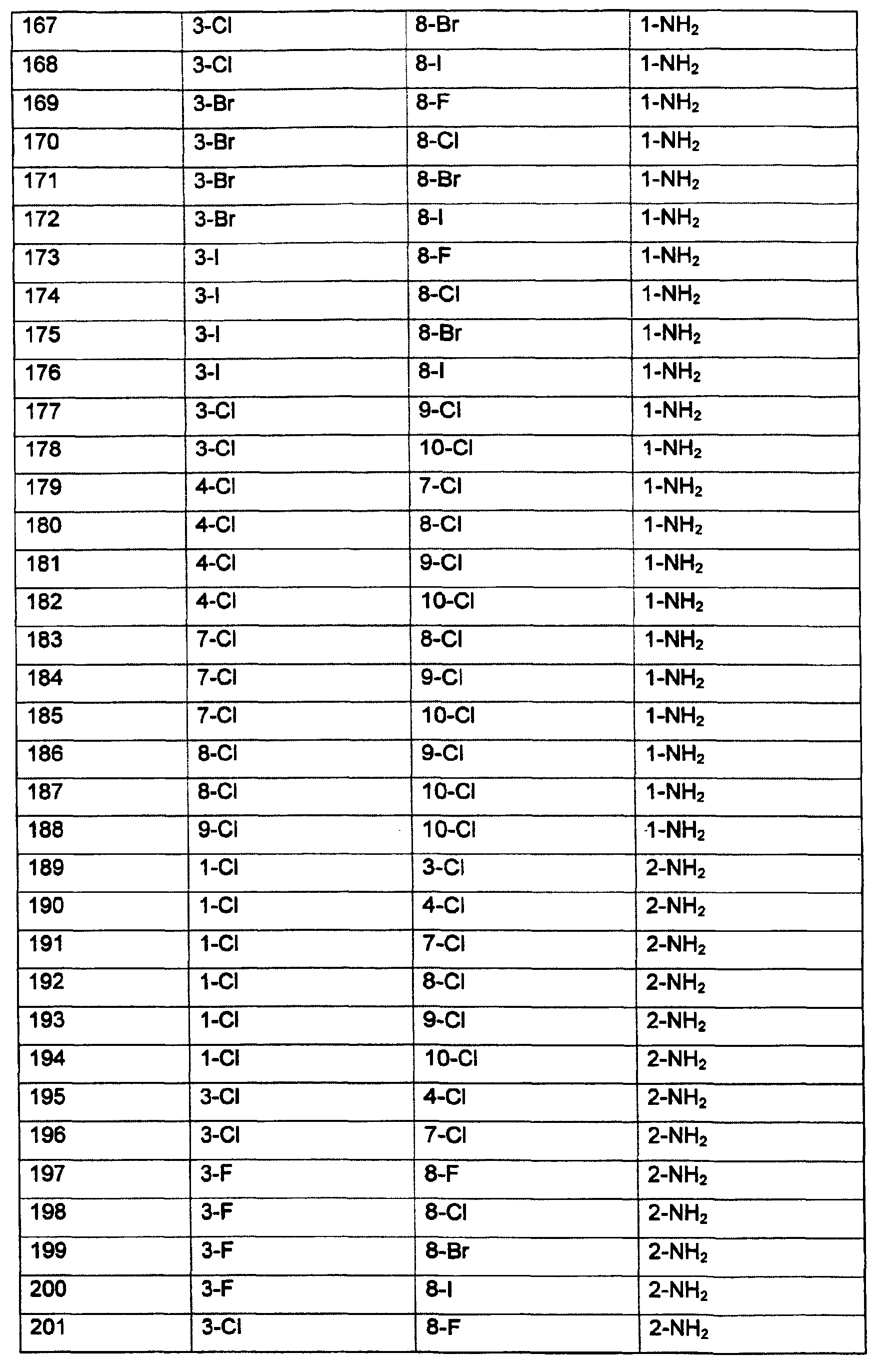

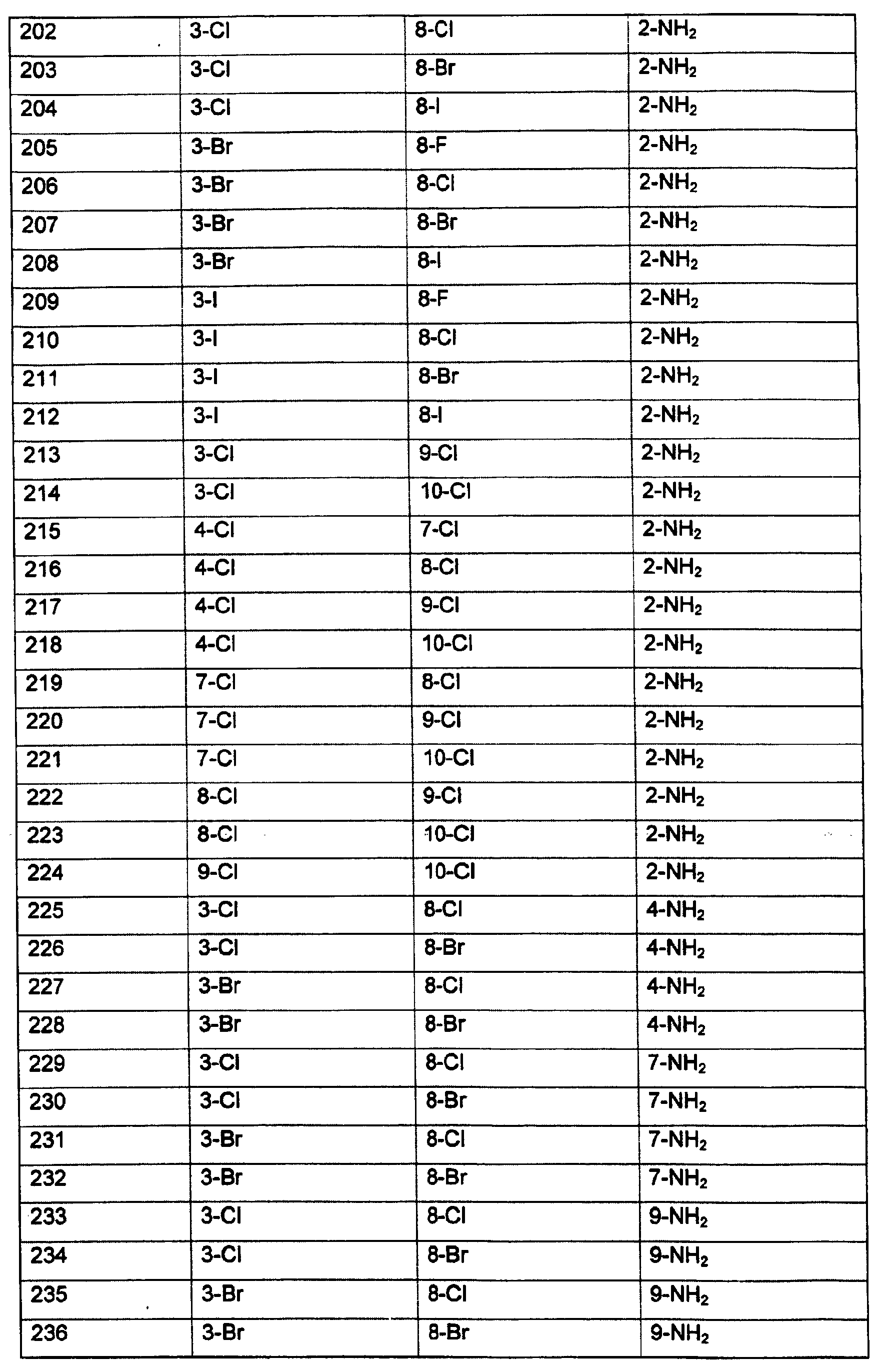

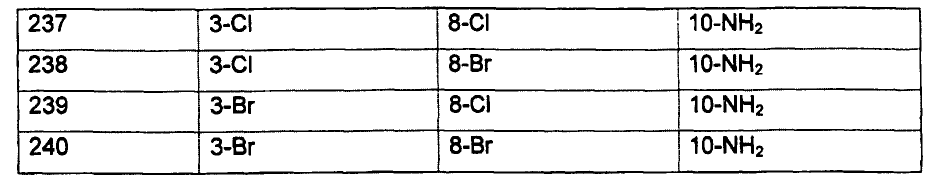

In the above table, preferred are Compound Nos. 1, 2, 3, 4, 5, 6, 7, 8, 33, 34, 37, 38, 39, 55, 56, 57, 61, 62, 65, 66, 161, 162, 163, 165, 166, 167, 169, 170, 171, 197, 198, 199, 201, 202, 203, 205, 206 and 207.

More preferred are Compound Nos. 1, 2, 3, 4, 6, 7, 8, 34, 38, 56, 57, 61, 65, 66, 166, 171, 202 and 207.

Particularly preferred are:
Compound No. 166: 1-amino-3,8-dichloro-6(5H)-phenanthridinone,
Compound No. 171: 1-amino-3,8-dibromo-6(5H)-phenanthridinone,
Compound No. 202: 2-amino-3,8-dichloro-6(5H)-phenanthridinone, and
Compound No. 207: 2-amino-3,8-dibromo-6(5H)-phenanthridinone.

The compound of formula (I), which is an active ingredient of the potentiator for neurotrophin effect of the present invention is known or can be prepared easily by a known manner [for example, WO 96/19458, WO 98/27975, US 5,589,483, Journal of Heterocyclic Chemistry, 7, 313 (1970), ibid., 7, 597 (1970), Australian Journal of Chemistry, 20, 2037(1967), Journal of Medicinal Chemistry, 12, 822 (1969), ibid., 37, 2085 (1994), Tetrahedron Letters, 36, 3911 (1968), Chemical Abstracts, 119, 48881 (1993), ibid., 118, 6533 (1993), ibid., 112, 181384(1990), ibid., 112, 168840 (1990), ibid., 112, 97946 (1990), ibid., 111, 23003 (1989), ibid., 107, 123664 (1987), ibid., 95, 42866 (1981), ibid., 95, 42867 (1981), ibid., 85, 46352 (1976), ibid., 72, 121337 (1970), ibid., 68, 59420 (1968), ibid., 83, 96982 (1975), ibid., 73, 35200 (1970), ibid., 71, 91236 (1969), etc.].

The compound of formula (I) serving as an active ingredient for the potentiator for neurotrophin effect of the present invention can be prepared easily in accordance with the following process.

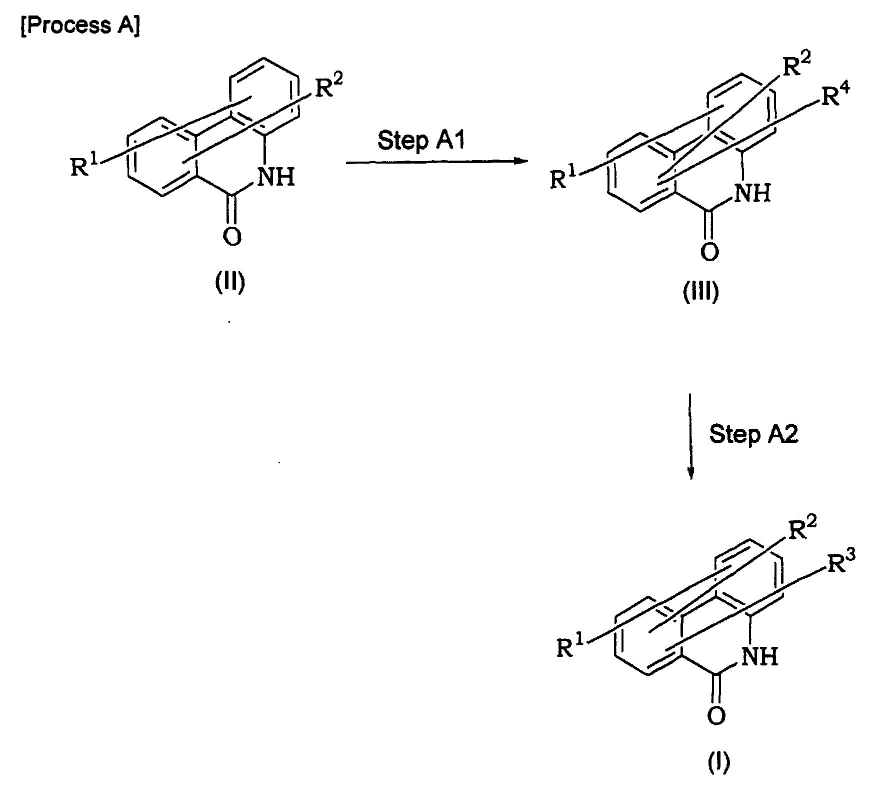

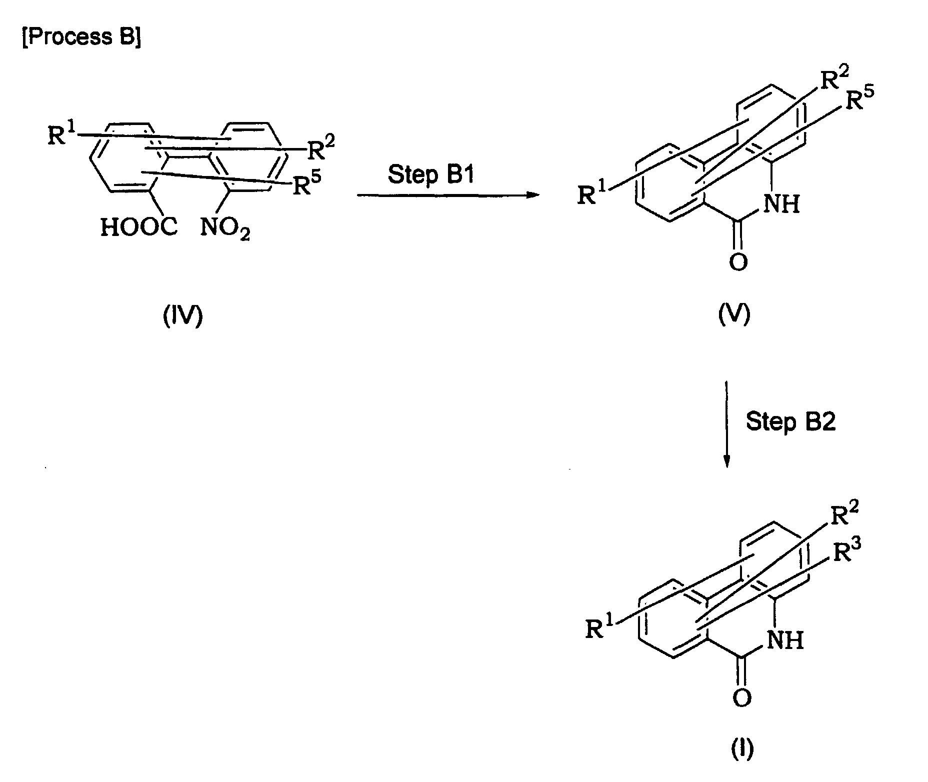

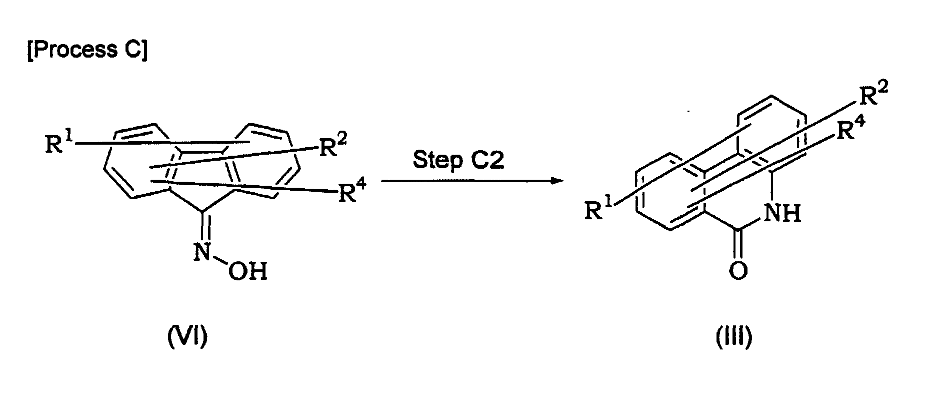

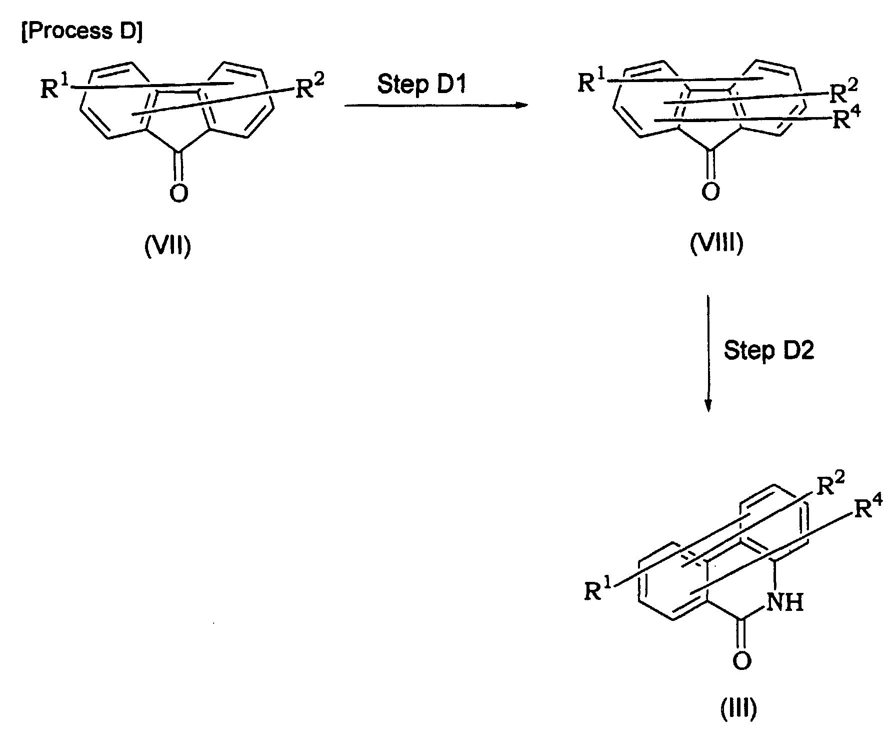

In the above-described formulae, R¹, R² and R³ have the same meanings as described above, R⁴ represents a nitro group and R⁵ represents a carboxyl group.

Process A is a process for preparing a compound of formula (I).

Step A1 is a step for preparing a compound of formula (III), by reacting a compound of formula (II) with nitric acid in acetic acid.

Although the reaction temperature differs depending on the nature of the starting material, it is usually -10°C to 150°C, preferably 0 to 100°C.

The reaction time differs depending on the nature of the starting material, the reaction temperature and the like, but it is usually 5 minutes to 12 hours, preferably 10 minutes to 3 hours.

After completion of the reaction, the desired product is collected from the reaction mixture in a conventional manner. For example, when the desired compound is a precipitate or the desired compound is precipitated by distilling off the solvent after completion of the reaction, the desired compound is available by collecting the precipitate by filtration; or adding water to the reaction mixture after completion of the reaction, extracting the desired compound by adding a solvent not miscible with water (eg. benzene, ether or ethyl acetate), washing the extracted organic layer with water, drying it over anhydrous magnesium sulfate and then distilling off the solvent. The desired compound can be further purified in a conventional manner, for example, recrystallization, reprecipitation or chromatography if necessary.

Step A2 is a step for preparing a compound of formula (I) by:
(1) reducing a compound of formula (III) by using palladium-carbon in the presence of formic acid in an inert solvent,
(2) reducing a compound of formula (III) by using tin chloride in the presence of concentrated hydrochloric acid in an inert solvent, or
(3) reducing a compound of formula (III) by using Raney nickel or palladium on carbon (preferably, palladium on carbon) in the presence of hydrazine (preferably, hydrazine hydrate) in an inert solvent.

There is no particular limitation on the solvent to be used in Step A2(1), Step A2(2) or Step A2(3) insofar as it has no adverse effect on the reaction and is capable of dissolving therein a starting substance to some extent. Examples of the solvent usable in Step A2(1) or Step A2(2) include alcohols such as methanol, ethanol, propanol, isopropanol, butanol and isobutanol, of which methanol and ethanol are preferred. Examples of the solvent usable in Step A2(3) include aliphatic hydrocarbons such as hexane, cyclohexane, heptane, ligroin and petroleum ether, aromatic hydrocarbons such as benzene, toluene and xylene, halogenated hydrocarbons such as dichloromethane, chloroform, carbon tetrachloride, 1,2-dichloroethane, chlorobenzene and dichlorobenzene, ethers such as diethyl ether, diisopropyl ether, tetrahydrofuran, dioxane, dimethoxyethane and di(ethylene glycol) dimethyl ether, and alcohols such as methanol, ethanol, propanol, isopropanol, butanol and isobutanol, and mixtures of the above-exemplified organic solvents. Of these, aromatic hydrocarbons and alcohols and mixtures of an aromatic hydrocarbon and an alcohol are preferred, of which the alcohols (particularly, ethanol) are more preferred.

Although the reaction temperature differs depending on the nature of the starting material, reagent and solvent, it is usually -20°C to 100°C (preferably, 20°C to 80°C) in Step A2(1), usually 0 to 150°C (preferably 50 to 100°C) in Step A2(2), and usually -20 to 150°C (preferably 20 to 50°C) in Step A2(3).

Although the reaction time differs depending on the nature of the starting material, reagent, solvent and reaction temperature, it is usually 10 minutes to 24 hours (preferably, 30 minutes to 6 hours) in Step A2(1), usually 10 minutes to 24 hours (preferably 30 minutes to 6 hours) in Step A2(2), and usually 10 minutes to 24 hours (preferably 30 minutes to 6 hours) in Step A2(3).

After completion of the reaction, the desired product of this step is collected from the reaction mixture in a conventional manner. For example, when insoluble matter is present, the desired compound may be obtained by filtering off the insoluble matter and concentrating the filtrate; or concentrating the filtrate, adding water to the residue, adjusting the pH of the solution to alkaline, adding a solvent not miscible with water (eg. benzene, ether or ethyl acetate) to extract the desired compound, washing the organic layer with water, drying it over anhydrous magnesium sulfate, and then distilling off the solvent. The desired compound can be further purified in a conventional manner, for example, by recrystallization, reprecipitation or chromatography if necessary.

Process (B) is another process for preparing the compound of formula (I).

Step B1 is a step for preparing a compound of formula (V) by reacting a compound of formula (IV) with Raney nickel in an aqueous solution of sodium carbonate (preferably, in a 10% aqueous solution of sodium carbonate) in the presence of hydrogen.

Although the reaction temperature differs depending on the nature of the starting material and the reagent, it is usually -10 to 100°C, preferably 0 to 50°C.

Although the reaction time differs depending on the nature of the starting material, reagent and the reaction temperature, it is usually 10 minutes to 10 hours, preferably 30 minutes to 5 hours.

After completion of the reaction, the desired product of this step is collected from the reaction mixture in a conventional manner. For example, the desired compound may be obtained by filtering off the catalyst after completion of the reaction, distilling off the solvent, adding water to the residue, acidifying the aqueous layer and then collecting the precipitate by filtration; or filtering off the catalyst after completion of the reaction, adjusting the pH of the reaction mixture to acidic using an acid (preferably, hydrochloric acid), adding a solvent not miscible with water (eg. benzene, ether or ethyl acetate) to extract the desired compound, washing the organic layer with water, drying it over anhydrous magnesium sulfate, and then distilling off the solvent. The desired compound can be further purified in a conventional manner, for example, by recrystallization, reprecipitation or chromatography if necessary.

Step B2 is a step for preparing a compound of formula (I) by reacting a compound of formula (V) with sodium azide in sulfuric acid.

Although the reaction temperature differs depending on the nature of the starting material, it is usually 0 to 150°C, preferably 50 to 100°C.

Although the reaction time differs depending on the nature of the starting material and the reaction temperature, it is usually 5 minutes to 6 hours, preferably 10 minutes to 1 hour.

After completion of the reaction, the desired product of this step is collected from the reaction mixture in a conventional manner. For example, the desired compound may be obtained by adding water to the reaction mixture, collecting the precipitate by filtration and treating the precipitate with an aqueous alkaline solution (preferably an aqueous solution of potassium hydroxide). The desired compound can be further purified in a conventional manner, for example, by recrystallization, reprecipitation or chromatography if necessary.

Process C is a process for preparing a compound of formula (III).

Step C1 is a step for preparing a compound of formula (III) by reacting a compound of formula (VI) with polyphosphoric acid.

Although the reaction temperature differs depending on the nature of the starting material, it is usually 0 to 150°C, preferably 50 to 100°C.

Although the reaction time differs depending on the nature of the starting material and the reaction temperature, it is usually 5 minutes to 6 hours, preferably 10 minutes to 1 hour.

After completion of the reaction, the desired product of this step is collected from the reaction mixture in a conventional manner. For example, when the desired compound is precipitated after the completion of the reaction or by distilling off the solvent, the desired compound may be obtained by collecting the precipitate by filtration; or by adding water to the reaction mixture after completion of the reaction, extracting the desired compound by adding a solvent not miscible with water (eg. benzene, ether or ethyl acetate), washing the extracted organic layer with water, drying it over anhydrous magnesium sulfate and then distilling off the solvent. The desired compound can be further purified in a conventional manner, for example, by recrystallization, reprecipitation or chromatography if necessary.

Process D is another process for preparing a compound of formula (III).

Step D1 is a step for preparing a compound of formula (VIII) by reacting a compound of formula (VII) with nitric acid in acetic acid. This step can be carried out under similar conditions to those described in Step A1.

Step D2 is a step for preparing a compound of formula (III) by reacting a compound of formula (VIII) with sodium azide in sulfuric acid. This step can be carried out under similar reaction conditions to those described in Step B2.

Starting materials of formulae (II), (IV), (VI) and (VII) are known or can easily be prepared by a known manner [for example, Synthesis, 4, 192 (1972), WO 96/19458, WO 98/27975, US 5,589,483, Journal of Heterocyclic Chemistry, 7, 313 (1970), ibid., 7, 597 (1970), Australian Journal of Chemistry, 20, 2037 (1967), Journal of Medicinal Chemistry, 7, 31 (1964), ibid., 12, 822 (1969), ibid., 37, 2085 (1994), Tetrahedron Letters, (36), 3911 (1968), etc.].

### Best Mode for Carrying out the Invention

The present invention will hereinafter be described in further detail by Examples and Formulation Examples. It should however be borne in mind that the scope of the present invention is not limited to or by them.

### Example 1

### Induction of differentiation in melanocytoma strain (PC12 cell) derived from rat adrenal medulla

PC12 cells were suspended in a DEM medium containing 10% equine serum and 5% bovine serum and the resulting suspension was charged in a collagen-coated 24-well plate. Twenty-four hours later, the medium was exchanged. A neurotrophin [human recombinant NGF or NT-3 (Austral Biologicals)] and 1-amino-3,8-dichloro-6(5H)-phenanthridinone (Compound No. 166) synthesized in accordance with the process described in Journal of Heterocyclic Chemistry, 7, 597 (1970), were added and cultured for 72 hours. After the medium was discarded, the cells were fixed with 2% glutaraldehyde and were observed under a microscope. The ratio of the cells having a longer outgrowth than the cell body was determined. The results are shown in Table 2.

**Table 2:**

| Ratio of PC12 cells having an outgrowth (%) | | | |
|---|---|---|---|
| Neurotrophin (ng/ml) | Compound-free | Compound No. 166 (3 µM) | Compound No. 166 (30 µM) |
| NGF(5) | 8 ± 0.9 | 12 ± 1.3 | 29 ± 2.6 |
| NGF (20) | 27 ± 2.8 | 51 ± 6.5 | 72 ± 2.0 |
| NGF (50) | 57 ± 3.6 | 79 ± 3.0 | 88 ± 0.7 |
| NT-3 (100) | 2 ± 0.6 | 62 ± 0.2 | 73 ± 3.6 |
| (expressed by mean ± standard deviation in the test of 3 cases) | | | |

NGF applied into the medium induced differentiation of PC12 cells and increased the ratio of the cell having neurite outgrowth. 1-Amino-3,8-dichloro-6(5H)-phenanthridinone, when applied with NGF, enhanced further the differentiation of the cell and further increased the ratio of the cell having the neurite outgrowth. Thus, the compound potentiated the NGF-induced differentiation and neurite outgrowth in PC12 cells. It has also been found that although NT-3 did not cause any sufficient differentiation-induction by itself when used alone, a combined application of NT-3 with 1-amino-3,8-dichloro-6(5H)-phenanthridinone markedly potentiated the differentiation-induction of PC12 cells. These results indicate that 1-amino-3,8-dichloro-6(5H)-phenanthridinone potentiated the effect of neurotrophin.

### Example 2

### Effects on survival of ganglion sensory neurons in dorsal spinal root ganglion of the rat spinal cord

The dorsal root ganglion of the spinal cord was isolated from fetuses of the SD rat (Nippon SLC) on 17 days definite pregnancy under a stereoscopic microscope. The ganglion was then treated with 1 mg/ml of collagenase, 2 mg/ml of trypsin and 2 mg/ml of DNase I, followed by dispersion in an MEM medium containing 10% bovine fetal serum. Immediately after the sensory neuron suspension was charged in a 12-well plate (Becton Dickinson Labware) which was coated in advance with laminin and poly-D-lysine, a neurotrophin (NGF or NT-3) and 1-amino-3,8-dichloro-6(5H)-phenanthridinone (Compound No. 166) or 6(5H)-phenanthridinone (Compound A) were added. When half of the medium was exchanged on the next day, cytosine arabinoside was added to the culture medium at a final concentration of 10 µM, in order to cause cell death other than to neurons. A neurotrophin and the test compound were added to the medium at their original concentrations. After culturing for a further two days, the neurons were photographed under a microscope and the number of viable neurons was counted. The results are summarized in Tables 3 and 4.

**[Table 3]**

| Number of viable sensory neurons in the dorsal root ganglion of the rat spinal cord (per mm²) | | |
|---|---|---|
| Neurotrophin (ng/ml) | Compound-free | Compound No. 166 (3 µM) |
| Neurotrophin-free | 5 ± 1.2 | 24 ± 1.6 |
| NGF (1 ng/ml) | 44 ± 5.5 | 71 ± 1.7 |
| NT-3 (100 ng/ml) | 19 ± 5.0 | 168 ± 21.1 |
| (each expressed as mean ± standard deviation calculated from 3 experiments) | | |

**[Table 4]**

| Number of viable sensory neurons in the dorsal root ganglion of the rat spinal cord (per mm²) | | | |
|---|---|---|---|
| Neurotrophin (ng/ml) | Compound -free | Compound No. 166 (1 µM) | Compound A (1 µM) |
| Neurotrophin-free | 3 ± 0.4 | 11 ± 4.0 | 6 ± 0.8 |
| NT-3 (100 ng/ml) | 33 ± 4.9 | 133 ± 13.1 | 37 ± 3.6 |
| [each expressed as mean ± standard deviation calculated from 3 experiments; Compound A: 6(5H)-phenanthridinone] | | | |

It has been found that 1-amino-3,8-dichloro-6(5H)-phenanthridinone markedly potentiated the effects of NGF or NT-3 which prolonged the survival time of sensory neurons in the dorsal root ganglion of the rat spinal cord, while 6(5H)-phenanthridinone had no effects. These results indicate that 1-amino-3,8-dichloro-6(5H)-phenanthridinone promotes the survival of peripheral sensory neurons.

### Example 3

### Enhancing action of neurotrophin response in hippocampal and strial tissue slices of matured rat

Male SD rats (Nippon SLC) were decapitated under anesthesia and the hippocampus and the corpus striatum were isolated. The tissue was cut into 0.2 x 0.2 mm slices with a "Mcllwain Tissue Chopper" (Mickle Laboratory Engineering Company), and were suspended in a DMEM medium. A neurotrophin (NGF or NT-3) and 1-amino-3,8-dichloro-6(5H)-phenanthridinone were added to the suspension and the mixture was kept at 37°C for 20 minutes. After washing the tissue with ice-cooled tris buffer physiological saline (TBS), the mixture was stirred vigorously and dissolved in TBS containing 1% Triton-X100, 10 µg/ml of leupeptin, 10 µg/ml of aprotinin, 1 mM of phenylmethylsulfonyl fluoride and 1 mM of sodium vanadate. After centrifugation at 14000 g for 15 minutes, the amount of protein in the supernatant was determined with Protein Assay Kit (Bio-Rad Laboratories, Ltd.). Anti-Trk antibody C-14 (Santa Cruz Biotechnology Inc) which specifically binds to Trk, a neurotrophin receptor, was added to the extract which was adjusted to contain an equal amount of protein and mixed at 4°C for 16 hours. Thus the Trk receptor in the tissue extract formed an immunocomplex with the antibody. The immunocomplex was precipitated with protein A Sepharose beads (Amersham Pharmacia Biotech). The Trk protein collected was separated from other proteins by sodium dodecyl sulfate-polyacrylamide gel electrophoresis and then transferred to a nitrocellulose membrane by Western blot assay. The membrane was reacted with Antibody 4G10 (Upstate Biotechnology Inc) which recognizes phosphotyrosine and the amount of phosphotyrosine of the Trk protein fixed on the membrane was visualized on a film by an enhanced chemiluminescence kit (Amersham Pharmacia Biotech). The film was photographed by a digital camera and the density of the band was determined by digital image analyzing software (Eastman Kodak Company). The results are shown in Table 5.

**[Table 5]**

| Amount of neurotrophin receptor Trk activated in matured rat cerebral tissues | | |
|---|---|---|
| Tissue | NT-3 (100 ng/ml) | NT-3 (100 ng/ml) + Compound No. 166 (30 µM) |
| Corpus striatum | 12 | 68 |
| Hippocampus | 27 | 58 |

Values are expressed relative to the amount (100) activated by NGF in the strial tissue.

From the results shown in Table 5, it was proved that 1-amino-3,8-dichloro-6(5H)-phenanthridinone has a neurotrophin enhancing action in the adult central nervous system which is a target of treatment for neurodegenerative diseases. Formulation Example 1

### Hard capsule

A capsule is obtained by mixing 50 mg of the compound of Example 7(d) in the powdery form, 128.7 mg of lactose, 70 mg of cellulose and 1.3 mg of magnesium stearate, sifting the mixture through a 60-mesh sieve and then filling a 250-mg No.3 capsule with the resulting powder.

### Formulation Example 2

### Tablet

A tablet, 200 mg in weight, is obtained by mixing 50 mg of the compound of Example 7(d) in the powdery form, 124 mg of lactose, 25 mg of cellulose and 1 mg of magnesium stearate and compressing the resulting mixture with a tableting machine. This tablet may be coated with sugar as needed.

### Industrial Applicability

A potentiator for neurotrophin effect of the present invention containing a 6(5H)-phenanthridinone derivative represented by the formula (I) or a pharmacologically acceptable salt thereof has excellent neurotrophin-action enhancing activity and has weak toxicity so that it is useful as a preventive or therapeutic agent for neurodegenerative diseases [a preventive or therapeutic agent (particularly, a therapeutic agent) for Alzheimer-type dementia, Parkinson's disease, amyotrophic lateral sclerosis, Huntington's chorea and peripheral sensory neuropathy (particularly, Alzheimer-type dementia), or a preventive agent for apoptosis of neurons after cerebral ischemia or a therapeutic agent for the sequelae of it].

When the potentiator for neurotrophin effect of the present invention is used as a preventive or therapeutic agent for the above-described diseases, it can be administered orally in the dosage form of a tablet, capsule, granule, powder or syrup or parenterally in the dosage form of an injection or suppository as is or in combination with a pharmacologically acceptable excipient or diluent.

The above-described formulations can be prepared by a known manner using additives. Examples of such additives include excipients (eg. organic excipients, for example, sugar derivatives such as lactose, sucrose, dextrose, mannitol and sorbitol, starch derivatives such as corn starch, potato starch, α-starch and dextrin, cellulose derivatives such as crystalline cellulose, gum arabic, dextran and pullulan; and inorganic excipients, for example, silicate derivatives such as light silicic anhydride, synthetic aluminum silicate, calcium silicate and magnesium aluminate metasilicate, phosphates such as calcium hydrogenphosphate, carbonates such as calcium carbonate, and sulfates such as calcium sulfate), lubricants (for example, stearic acid, metal salts of stearic acid such as calcium stearate and magnesium stearate, talc, colloidal silica, waxes such as bees wax and spermaceti, boric acid, adipic acid, sulfates such as sodium sulfate, glycol, fumaric acid, sodium benzoate, DL-leucine, lauryl sulfates such as sodium lauryl sulfate and magnesium lauryl sulfate, silicic acids such as silicic anhydride and silicic hydrate and the above-exemplified starch derivatives), binders (for example, hydroxypropyl cellulose, hydroxypropylmethyl cellulose, polyvinyl pyrrolidone, macrogol and compounds similar to those exemplified above as excipients), disintegrants (for example, cellulose derivatives such as low-substituted hydroxypropyl cellulose, carboxymethyl cellulose, calcium carboxymethyl cellulose and internally crosslinked sodium carboxymethyl cellulose and chemically modified starches or celluloses such as carboxymethyl starch, sodium carboxymethyl starch; and crosslinked polyvinyl pyrrolidone), emulsifiers (for example, colloidal clay such as bentonite and veegum, metal hydroxides such as magnesium hydroxide and aluminum hydroxide, anionic surfactants such as sodium lauryl sulfate and calcium stearate, cationic surfactants such as benzalkonium sulfate and nonionic surfactants such as polyoxyethylene alkyl ethers, polyoxyethylene sorbitan fatty acid esters and sucrose fatty acid esters), stabilizers (parahydroxybenzoates such as methyl paraben and propyl paraben, alcohols such as chlorobutanol, benzyl alcohol and phenyl ethyl alcohol, benzalkonium chloride, phenols such as phenol and cresol, thimerosal, dehydroacetic acid, and sorbic acid), taste and odor corrigents (ordinarily-used sweeteners, souring agents and flavors), and diluents.

The dose differs depending on the symptoms, age and the like of the patient, but is, per adult, 1 mg/unit dose as the lower limit (preferably, 10 mg/unit dose) and 1000 mg/unit dose as the upper limit (preferably, 500 mg/unit dose) in the case of oral administration, while it is 0.5 mg/unit dose as the lower limit (preferably, 5 mg/unit dose) and 500 mg/unit dose as the upper limit (preferably, 250 mg/unit dose) in the case of intravenous administration. The dose is desirably administered in 1 to 6 portions a day.

## Claims

1. A potentiator for neurotrophin effect comprising a 6(5H)-phenanthridinone derivative represented by the following formula (I): 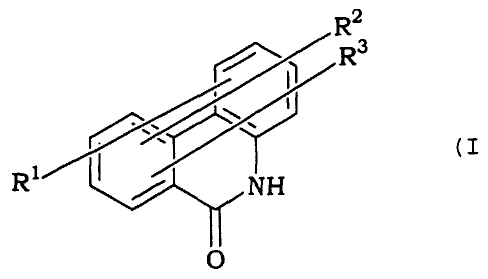 (wherein, R¹ and R² are the same or different and each independently represents a hydrogen atom or a halogen atom, and R³ represents an amino group) or a pharmacologically acceptable salt thereof.

2. A potentiator for neurotrophin effect according to claim 1, wherein R¹ and R² are the same or different and each independently represents a hydrogen atom, a chlorine atom or a bromine atom.

3. A potentiator for neurotrophin effect according to claim 1, wherein R¹ and R² each independently represents a chlorine atom or a bromine atom.

4. A potentiator for neurotrophin effect according to claim 1, selection from 1-amino-3,8-dichloro-6(5H)-phenanthridinone, 1-amino-3,8-dibromo-6(5H)-phenanthridinone, 2-amino-3,8-dichloro-6(5H)-phenanthridinone and 2-amino-3,8-dibromo-6(5H)-phenanthridinone, or a pharmacologically acceptable salt thereof.

5. A preventive or therapeutic agent for a neurodegenerative disease, which comprises a potentiator for neurotrophin effect as claimed in any one of claims 1 to 4.

6. A preventive or therapeutic agent according to claim 5, wherein the neurodegenerative disease is Alzheimer-type dementia, Parkinson's disease, amyotrophic lateral sclerosis, Huntington's chorea or peripheral sensory neuropathy.

7. A preventive or therapeutic agent according to claim 5, wherein the neurodegenerative disease is apoptosis of neurons after cerebral ischemia or a sequela thereof.
